(19)

Europäisches Patentamt
European Patent Office
Office européen des brevets

(11) EP 0 708 082 B1

(12) EUROPÄISCHE PATENTSCHRIFT

(45) Veröffentlichungstag und Bekanntmachung des Hinweises auf die Patenterteilung:
**07.04.1999 Patentblatt 1999/14**

(51) Int Cl.$^6$: **C07C 249/04**, C07C 251/38, C07C 251/40

(21) Anmeldenummer: **95116246.0**

(22) Anmeldetag: **16.10.1995**

(54) **Verfahren zur Herstellung von O-substituierten Hydroxylammoniumsalzen**

Process for preparing O-substituted hydroxylammonium salts

Procédé pour la préparation des sels d'hydroxylammonium O-substitué

(84) Benannte Vertragsstaaten:
**AT BE CH DE ES FR GB IT LI NL**

(30) Priorität: **22.10.1994 DE 4437905**

(43) Veröffentlichungstag der Anmeldung:
**24.04.1996 Patentblatt 1996/17**

(60) Teilanmeldung: **98115428.9 / 0 885 878**

(73) Patentinhaber: **BASF AKTIENGESELLSCHAFT**
**67056 Ludwigshafen (DE)**

(72) Erfinder:
- **Keil, Michael, Dr.**
  **D-67251 Freinsheim (DE)**
- **Wahl, Josef**
  **D-67105 Schifferstadt (DE)**
- **Klein, Ulrich, Dr.**
  **D-67117 Limburgerhof (DE)**
- **Will, Wolfgang, Dr.**
  **D-67281 Kirchheim (DE)**

(56) Entgegenhaltungen:
**EP-A- 0 158 159** **EP-A- 0 591 798**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 708 082 B1

Printed by Jouve, 75001 PARIS (FR)

## Beschreibung

Die vorliegende Erfindung betrifft ein Verfahren zur Herstellung von O-substituierten Hydroxylammoniumsalzen der Formel I

$$H_2NOR \times HX \qquad I$$

in der R einen $C_1$-$C_6$-Alkyl- oder $C_2$-$C_6$-Alkenylrest, jeweils ggf. halogensubstituiert bedeutet und X für Chlor oder Brom steht.

Die Verbindungen I stellen wertvolle Zwischenprodukte zur Herstellung von Arzneimittel- und Pflanzenschutzwirkstoffen dar. Solche Wirkstoffe sind beispielsweise in EP-A 253 213 beschrieben.

Die Herstellung von Hydroxylammoniumsalzen über Einzelstufen ist im Prinzip bekannt. Nach Verknüpfung von Hydroxylamin mit einer Schutzgruppe A erhält man zweifach am N-substituierte Hydroxylamine. Diese werden anschließend am Sauerstoffatom elektrophil substituiert und in einem dritten Schritt mit Mineralsäuren unter Freisetzung des Schutzgruppenrestes zu den Verbindungen I gespalten:

$$H_2NOH \rightarrow A{=}NOH$$

$$A{=}NOH + RY \rightarrow A{=}NOR$$

$$A{=}NOR + HX \rightarrow H_2NOR \times HX$$

(R z.B. Alkyl, X und Y z.B. Cl oder Br)

Die einzelnen Stufen sind anhand von Beispielen u.a. in Houben-Weyl, Methoden der Organ. Chem., Bd. 10/1, Seiten 1181 ff (4. Aufl., 1971) und Bd. 10/4, Seiten 55 ff 4. Auflage, 1968 beschrieben. So kann die Schutzgruppe A beispielsweise ein Phthaloylrest, zwei Sulfonatreste oder ein 2-Propyliden-Rest sein. Die Herstellung von Acetonoxim gemäß der ersten Verfahrensstufe ist beispielsweise in Houben-Weyl, Bd. 10/4, S. 58 zitiert und erfolgte in wäßrigem Medium. Die Alkylierung von Acetonoxim ist beispielsweise beschrieben in Houben Weyl, Bd. 10/4, S. 220 ff, sowie in EP-A 23 560, EP-A 121 701, EP-A 158 159.

Eine unerwünschte Nebenreaktion bei diesen Reaktionsbedingungen ist dabei die N-Methylierung, die zu Nitronen führt und somit die Ausbeuten an O-substituierten Produkten erheblich mindert (Houben-Weyl, Bd. 10/4, S. 220).

Die Spaltung von Acetonoximmethylether mit Mineralsäuren ist beispielsweise in Houben-Weyl, Bd. 10/1, S. 1186 ff. beschrieben. Die Reaktion gelingt jedoch in zufriedenstellenden Ausbeuten nur, wenn Aceton destillativ aus dem Gleichgewicht gezogen wird (EP-A 259 850, EP-A 591 798).

Die Umsetzung der beschriebenen drei Einzelstufen zu einem wirtschaftlichen Prozeß in der Produktion von großen Mengen eines Alkoxyamins beinhaltet jedoch folgende Schwierigkeiten:

1. Die Isolierung und Reinigung der Zwischenstufen durch Filtration oder Destillation ist aufwendig und teuer. Die Gesamtausbeute über alle Stufen ist unbefriedigend.

2. Die bei der Alkylierung normalerweise zur Verwendung kommenden polaren bzw. protischen Lösungsmittel (siehe EP-A 23 560 und 121 701) stören bei der anschließenden Hydrolyse und müssen demgemäß von den O-substituierten Oximen vollständig abgetrennt und zurückgeführt werden. Bei Verwendung von nicht polaren aprotischen Lösungsmitteln wie Toluol, Xylol, Cyclohexan in Stufe 2 muß gemäß EP-A 158 159 ein großer Überschuß an Oxim eingesetzt werden, der dann umständlich wieder isoliert werden muß.

Es besteht daher Bedarf an einem einfachen Verfahren, bei dem die oben geschilderten Nachteile vermieden werden. Verbunden mit der Realisierung dieser Anforderungen wäre ein geringerer Apparateaufwand, eine günstigere Gesamtausbeute und damit eine höhere Wirtschaftlichkeit.

Es wurde nun ein Verfahren zur Herstellung von O-substituierten Hydroxylammoniumsalzen der Formel I gefunden, das dadurch gekennzeichnet ist, daß man in einem integrierten Prozeß, ohne Isolierung von Zwischenstufen

a) Aceton mit Hydroxylammoniumsulfat und Natronlauge zum Acetonoxim der Formel II

umsetzt;

b) die so erhaltene Lösung von Acetonoxim mit Natronlauge versetzt und vollständig Wasser auskreist,

c) die so erhaltene Suspension des Acetonoxim-Na-salzes III,

mit Alkylierungsmitteln der Formel IV

$$RY \qquad IV$$

worin R die in Formel I genannte Bedeutung hat und Y für eine nukleofuge Abgangsgruppe steht, bei Drucken von 0,5 bis 15 bar und Temperaturen bis 140°C zu Acetonoximethern der Formel V

umsetzt;

d) die Acetonoximether mit Säuren HX zu den Produkten I spaltet

wobei man in allen Verfahrensstufen a) bis d) ein einheitliches, nichtpolares aprotisches Lösungsmittel verwendet.

Nach dem erfindungsgemäßen Verfahren werden alle Reaktionsschritte im Eintopfverfahren in Gegenwart eines organischen nicht-protischen Lösungsmittels, das besonders als Schleppmittel zum Auskreisen von Reaktionswasser geeignet ist, vorgenommen.

Besonders vorteilhaft kommen aromatische, aliphatische und cycloaliphatische Kohlenwasserstoffe wie z.B. Benzol, Toluol, o-, m- oder p-Xylol, Hexan oder Cyclohexan in Betracht. Besonders bevorzugt ist Toluol.

Die einzelnen Schritte des Gesamtverfahrens sind im folgenden Reaktionsschema zusammengefaßt:

$$2\ (CH_3)_2C{=}O + (H_2NOH)_2 \times H_2SO_4 + 2\ NaOH \xrightarrow{-H_2O} (CH_3)_2C{=}N{-}OH\ (II) + Na_2SO_4$$

$$(CH_3)_2C{=}N{-}OH\ (II) + NaOH + RY\ (IV) \longrightarrow (CH_3)_2C{=}N{-}OR\ (V) + NaY$$

$$(CH_3)_2C{=}N{-}OR\ (V) + HX + H_2O \longrightarrow H_2NOR \times HX\ (I) + (CH_3)_2C{=}O$$

Zunächst setzt man Hydroxylammoniumsulfat mit Aceton und Natronlauge zum Acetonoxim II um, versetzt mit Natronlauge, entfernt azeotrop das Reaktionswasser und alkyliert zum Acetonoximether V. Dieser wird ohne weitere Reinigung mit Säuren HX zu den gewünschten Ammoniumsalzen I gespalten.

Der erste Reaktionschritt wird in an sich bekannter Weise in wäßrigem Medium durchgeführt und dann das Oxim II mit dem Lösungsmittel extrahiert. Ohne weitere Reinigung wird die Produktlösung mit etwa äquimolaren Mengen an Natronlauge, z.B. 0,8 bis 1,2 mol, insbesondere 1,0 mol, bezogen auf II, Natronlauge versetzt, so daß sich das Natriumsalz III bildet, azeotrop Toluol/Wasser ausgekreist und die toluolische Suspension des Natriumsalzes von Acetonoxim mit dem Alkylierungsmittel RY umgesetzt. Als Alkylierungsmittel können vorteilhaft $C_1$-$C_6$-Alkyl- bzw. $C_2$-$C_6$-Alkenylhalogenide, insbesondere Chloride oder Bromide verwendet werden. Daneben kommen auch Dialkylsulfate wie Dimethylsulfat in Betracht. Für die Herstellung von Ammoniumsalzen mit R=Methyl wird vorzugsweise Methylchlorid als Alkylierungsmittel verwendet.

Das Alkylierungsmittel kann nach den im Stand der Technik beschriebenen Mengenangaben verwendet werden. Ein Überschuß an Oximsalzen gegenüber dem Alkylierungsmittel ist allerdings nach dem erfindungsgemäßen Verfahren nicht erforderlich.

Die Alkylierung zum Acetonoximether V wird bei Temperaturen bis 140°C, z.B. 20 bis 140°C, insbesondere 30 bis 80°C und einem Druck von 0,5 bis 15 bar, insbesondere 1 bis 4 bar durchgeführt.

Die Alkylierung kann gegebenenfalls unter Zusatz von katalytischen Mengen Phasentransferkatalysatoren wie Tetraalkylammonium-halogeniden oder quarternären Phosphoniumsalzen erfolgen, die einen reaktionsbeschleunigenden Einfluß haben, durchgeführt werden. Beispiele für solche Salze sind Tetrabutylammoniumbromid, Benzyltrimethylammoniumchlorid oder Tetrabutylphosphoniumbromid.

Nach wäßriger Extraktion wird der Oximether V direkt im organischen Lösungsmittel, z.B. Toluol mit konzentrierten wäßrigen Säuren von HBr bzw. insbesondere Salzsäure gespalten, wobei gleichzeitig ein Gemisch von Aceton, dem Lösungsmittel und verdünnter HX abdestilliert wird. Das Produkt $H_2NOR$ x HX bleibt am Ende der Reaktion gelöst in Wasser und kann für Folgereaktionen direkt weiter eingesetzt oder mit üblichen Methoden isoliert werden.

Nach dem erfindungsgemäßen Verfahren werden Ammoniumsalze I mit R=$C_1$-$C_6$-Alkyl oder $C_2$-$C_6$-Alkenyl jeweils ggf. halogensubstituiert hergestellt. Aufgrund der Weiterverarbeitung zu biologisch wirksamen Verbindungen sind als R insbesondere $C_1$-$C_4$-Alkyl- und $C_2$-$C_4$-Alkenylreste ggf. halogensubstituiert, z.B. 1- bis 3-fach durch Fluor, Chlor oder Brom substituiert und vor allem R=Methyl bevorzugt.

Das folgende Beispiel verdeutlicht das beanspruchte Verfahren. Die erzielte Gesamtausbeute ist überraschend hoch, die Versuchsdurchführung ist wesentlich einfacher als die Maßnahmen bei schrittweiser Herstellung der Zwischenstufen.

Beispiele

Herstellung von Methoxyaminhydrochlorid ohne Isolierung von Zwischenstufen

Beispiel 1

980 ml Wasser, 390 g Toluol, 328 g Hydroxylammoniumsulfat, 320 g 50 %ige Natronlauge und 232 g Aceton werden bei pH 5 ca. 1 h gerührt. Die wäßrige Phase wird noch 2 x mit 460 ml Toluol extrahiert und die Toluolextrakte zur Toluolphase gegeben. Nach Zugabe von 320 g konz. NaOH kreist man 250 g Wasser aus. Die erhaltene Kristallmaische wird bei 50°C unter 2 bar Druck mit 210 g Methylchlorid versetzt und 5 h bei 50°C intensiv gerührt. Nach Extraktion mit 800 g Wasser wird die toluolische Phase mit 592 g konz. Salzsäure versetzt und unter Rückfluß Toluol/Aceton/Salzsäure abdestilliert. Nach 8 Stunden wird abgekühlt und die Toluolphase abgetrennt. Die wäßrige Phase enthält 238 g Methoxyaminhydrochlorid = 71 % Ausbeute ber. Aceton (über alle Stufen).

Beispiel 2

980 ml Wasser, 390 g Toluol, 328 g Hydroxylammoniumsulfat, 320 g 50 %ige Natronlauge und 232 g Aceton werden bei pH5 ca. 1 h gerührt. Die wäßrige Phase wird noch 2x mit je 460 ml Toluol extrahiert und die Toluolextrakte zur Toluolphase gegeben. Nach Zugabe von 320 g konz. NaOH kreist man 250 g Wasser aus. Die erhaltene Kristallmaische wird mit 1,5 g Tetrabutylammoniumbromid versetzt und 5 h bei 50°C unter max. 2 bar Druck mit 210 g Methylchlorid versetzt und 5 h bei 50°C intensiv gerührt. Nach Extraktion mit 800 g Wasser wird die toluolische Phase mit 750 g konz. Salzsäure extrahiert und der erhaltene saure Extrakt bei max. 100°C Blasentemperatur destilliert. Nach vollständigem Abdestillieren von Aceton wird mit 400 g Wasser verdünnt. Die erhaltene 30 %ige Lösung enthält 230 g Methoxyaminhydrochlorid = 69 % Ausbeute ber. Aceton.

Vergleichende Betrachtung zu Literaturausbeuten

Die Herstellung von Acetonoxim gelingt mit ca. 90 % Ausbeute. Acetoxim-O-methylether ist optimiert laut EP-A 23 560 in 62 % Ausbeute erhältlich. Die Spaltung zu Methoxyaminhydrochlorid gelingt nach EP-A 591 798 in 90 % Ausbeute. Die Gesamtausbeute beträgt nach Multiplikation der Einzelausbeuten nur 50 %.

## Patentansprüche

1. Verfahren zur Herstellung von O-substituierten Hydroxylammoniumsalzen der Formel I

$$H_2NOR \times HX \qquad \text{I}$$

in der R einen $C_1$-$C_6$-Alkyl- oder $C_2$-$C_6$-Alkenylrest, jeweils ggf. halogensubstituiert bedeutet und X für Chlor oder Brom steht, dadurch gekennzeichnet, daß man in einem integrierten Prozeß, ohne Isolierung von Zwischenstufen

a) Aceton mit Hydroxylammoniumsulfat und Natronlauge zum Acetonoxim der Formel II

$$(H_3C)_2C{=}N{-}OH \qquad \text{II}$$

umsetzt;

b) die so erhaltene Lösung von Acetonoxim mit Natronlauge versetzt und vollständig Wasser auskreist,

c) die so erhaltene Suspension des Acetonoxim-Na-salzes III

N
O⊖ Na⊕
C
H3C
CH3
III

mit Alkylierungsmitteln der Formel IV

RY IV

worin R die in Formel I genannte Bedeutung hat und Y für eine nukleofuge Abgangsgruppe steht, bei Drucken von 0,5 bis 15 bar und Temperaturen bis 140°C gegebenenfalls unter Zusatz von Phasentransferkatalysatoren zu Acetonoximethern der Formel V

N
OR
C
H3C
CH3
V

umsetzt,
wobei kein Überschuß an III gegenüber IV eingesetzt wird;

d) die Acetonoximether mit Säuren HX zu den Produkten I spaltet,

wobei man in allen Verfahrensstufen a) bis d) ein einheitliches, nichtpolares aprotisches Lösungsmittel verwendet.

**2.** Verfahren nach Anspruch 1, dadurch gekennzeichnet, daß man als Lösungsmittel Toluol, Xylol, Hexan oder Cyclohexan verwendet.

**3.** Verfahren nach Anspruch 1, dadurch gekennzeichnet, daß man als Lösungsmittel Toluol verwendet.

**4.** Verfahren nach Anspruch 1, dadurch gekennzeichnet, daß man als Alkylierungsmittel Methylchlorid verwendet.

**5.** Verfahren nach Anspruch 1, dadurch gekennzeichnet, daß man als Phasentransferkatalysator Tetrabutylammoniumbromid verwendet.

**6.** Verfahren nach Anspruch 1, dadurch gekennzeichnet, daß man als Säure HX konzentrierte Salzsäure verwendet.

**7.** Verfahren nach Anspruch 1, dadurch gekennzeichnet, daß man das Acetonoxim II mit 0,8 bis 1,2 mol-Äquivalenten an Natronlauge zum Natriumsalz III umsetzt.

**8.** Verfahren nach Anspruch 1, dadurch gekennzeichnet, daß man die Alkylierung des Acetonoxim-Na-salzes III mit dem Alkylierungsmittel IV bei Drucken von 1 bis 4 bar durchführt.

## Claims

**1.** A process for preparing O-substituted hydroxylammonium salts of the formula I

$$H_2NOR \times HX \qquad I$$

where R is a $C_1$-$C_6$-alkyl or $C_2$-$C_6$-alkenyl radical, each of which may be halogen-substituted, and X is chlorine or bromine, which comprises reacting in an integrated process, without isolation of intermediates

a) acetone with hydroxylammonium sulfate and sodium hydroxide solution to give the acetone oxime of the formula II

b) treating the solution of acetone oxime thus obtained with sodium hydroxide solution and completely removing water,

c) reacting the suspension thus obtained of the acetone oxime Na salt III

with alkylating agents of the formula IV

$$RY \qquad IV$$

where R has the meaning mentioned in formula I and Y is a nucleofugic leaving group, at from 0.5 to 15 bar and at up to 140°C, if appropriate with addition of phase-transfer catalysts, to give acetone oxime ethers of the formula V

no excess of III in relation to IV being employed;

d) cleaving the acetone oxime ethers with acids HX to give the products I,

a homogeneous, nonpolar aprotic solvent being used in all process steps a) to d).

**2.** A process as claimed in claim 1, wherein the solvent used is toluene, xylene, hexane or cyclohexane.

**3.** A process as claimed in claim 1, wherein the solvent used is toluene.

**4.** A process as claimed in claim 1, wherein the alkylating agent used is methyl chloride.

**5.** A process as claimed in claim 1, wherein the phase-transfer catalyst used is tetrabutylammonium bromide.

**6.** A process as claimed in claim 1, wherein the acid HX used is concentrated hydrochloric acid.

**7.** A process as claimed in claim 1, wherein the acetone oxime II is reacted with from 0.7 to 1.2 molar equivalents of sodium hydroxide solution to give the sodium salt III.

**8.** A process as claimed in claim 1, wherein the alkylation of the acetone oxime Na salt III with the alkylating agent IV is carried out at from 1 to 4 bar.

**Revendications**

**1.** Procédé pour la préparation de sels d'hydroxylammonium substitués à l'oxygène et répondant à la formule I

$$H_2NOR \times HX \qquad \text{I}$$

dans laquelle R représente un groupe alkyle en C1-C6 ou alcényle en C2-C6 portant chacun éventuellement des substituants halogéno, et X représente le chlore ou le brome, caractérisé par le fait que, dans des opérations intégrées, sans isolement des produits intermédiaires

a) on fait réagir l'acétone avec le sulfate d'hydroxylammonium et la lessive de soude, pour formation de l'acétonoxime de formule II

OH
N
C
H3C
CH3
II


b) à la solution d'acétonoxime ainsi obtenue, on ajoute de la lessive de soude et on élimine totalement l'eau, ce qui donne une suspension du sel de sodium de l'acétonoxime III

O⊖ Na⊕
N
C
H3C
CH3
III


c) qu'on convertit, par réaction avec les agents alkylants de formule IV

$$RY \qquad \text{IV}$$

dans laquelle R a les significations indiquées en référence à la formule I et Y représente un groupe éliminable nucléofuge, à des pressions de 0,5 à 15 bar et des températures allant jusqu'à 140°C, le cas échéant avec adjonction de catalyseurs à transfert de phase, en évitant d'utiliser un excès de III par rapport à IV, en éthers de l'acétonoxime de formule V

OR
N
C
$H_3C$
$CH_3$

V

d) qu'on convertit en les produits I par scission à l'aide d'acides HX,

en utilisant dans tous les stades opératoires a) à d) un solvant homogène aprotonique non polaire.

**2.** Procédé selon la revendication 1, caractérisé par le fait que le solvant utilisé est le toluène, le xylène, l'hexane ou le cyclohexane.

**3.** Procédé selon la revendication 1, caractérisé par le fait que le solvant utilisé est le toluène.

**4.** Procédé selon la revendication 1, caractérisé par le fait que l'agent alkylant utilisé est le chlorure de méthyle.

**5.** Procédé selon la revendication 1, caractérisé par le fait que le catalyseur à transfert de phase utilisé est le bromure de tétrabutylammonium.

**6.** Procédé selon la revendication 1, caractérisé par le fait que l'acide HX est l'acide chlorhydrique concentré.

**7.** Procédé selon la revendication 1, caractérisé par le fait que l'on fait réagir l'acétonoxime II avec 0,8 à 1,2 équivalent molaire de lessive de soude pour formation du sel de sodium III.

**8.** Procédé selon la revendication 1, caracétisé par le fait que l'alkylation du sel de sodium d'acétonoxime III par l'agent alkylant IV est réalisée sous des pressions de 1 à 4 bar.